# EUROPEAN PATENT SPECIFICATION

(11) **EP 4 397 648 B1**
(45) Date of publication and mention of the grant of the patent: **05.11.2025**
(21) Application number: 23863412.5
(22) Date of filing: 01.09.2023
(51) Int. Cl.: C07C 29/14, C07C 29/74, C07C 31/20, C07C 29/141, C07C 45/75, C07C 45/80, C07C 45/82

(54) **METHOD FOR PREPARING NEOPENTYL GLYCOL**
VERFAHREN ZUR HERSTELLUNG VON NEOPENTYLGLYKOL
PROCÉDÉ DE PRÉPARATION DE NÉOPENTYLGLYCOL

(30) Priority: 08.09.2022 KR 20220114485; 25.08.2023 KR 20230111966
(43) Date of publication of application: 10.07.2024
(73) Proprietor: LG Chem, Ltd., Yeongdeungpo-gu Seoul 07336 (KR)
(72) Inventor: KIM, Sung Kyun, Daejeon 34122 (KR); LEE, Sung Kyu, Daejeon 34122 (KR); HA, Na Ro, Daejeon 34122 (KR); CHO, Seung Sik, Daejeon 34122 (KR)
(74) Representative: Goddar, Heinz J.
(86) International application number: PCT/KR2023/013048
(87) International publication number: WO 2024/053936

(56) References cited:
- EP-A1- 3 219 698
- CN-A- 111 393 260
- KR-A- 20160 133 843
- KR-A- 20160 133 843
- KR-A- 20170 029 311
- KR-B1- 100 343 797
- KR-B1- 100 852 283

## Description

### [Technical Field]

### Technical Field

The present invention relates to a method for preparing neopentyl glycol, and more particularly, to a method for recovering and recycling an unreacted raw material and a catalyst of an aldol condensation reaction.

### [Background Art]

Neopentyl glycol may be generally prepared by performing an aldol condensation reaction of isobutyl aldehyde and formaldehyde in the presence of a catalyst to form hydroxypivaldehyde and proceeding a hydrogenation reaction of the hydroxypivaldehyde.

However, formic acid is produced from a Cannizzaro side reaction occurring in the aldol condensation reaction, the catalyst is changed into a catalyst salt by the formic acid, and the catalyst salt is in the form of an aqueous phase and conventionally treated as waste water.

That is, an environmental pollution problem is caused by the discarded catalyst salt, and as an added catalyst is produced as a catalyst salt and discarded, production costs increase during a process of continuously adding a new catalyst.

In addition, when unreacted isobutyl aldehyde in the aldol condensation reaction and a catalyst salt produced by a side reaction are recovered by separate processes, respectively, after the aldol condensation reaction process, process management is inefficient and energy usage is excessively consumed. Therefore, a process which is environmentally friendly and may further decrease energy usage in recovering and reusing unreacted isobutyl aldehyde and a discarded catalyst salt should be introduced. EP3219698A1 relates to an economical apparatus for preparing glycol which reduces loss of a raw material and provides a high neopentyl glycol yield while inhibiting generation of by-products, and a method of preparing the same.

### [Disclosure]

### [Technical Problem]

In order to solve the problems mentioned in the Background Art, an object of the present invention is to provide a method for preparing neopentyl glycol, which is environmentally friendly in the entire process and may further reduce energy usage while obtaining high-purity neopentyl glycol with a high recovery rate.

### [Technical Solution]

In one general aspect, provided is a method for preparing neopentyl glycol that includes: performing an aldol condensation reaction of a formaldehyde aqueous solution and isobutyl aldehyde in the presence of a catalyst in an aldol reactor to obtain a first reaction product including hydroxypivaldehyde; supplying the first reaction product to an aldol extraction column and bringing the first reaction product into contact with an extractant to obtain an extract including hydroxypivaldehyde and a raffinate including a catalyst salt; supplying the raffinate to a saponification reactor to reduce the catalyst salt to the catalyst and supplying a discharge stream from the saponification reactor including the catalyst to an integrated recovery column; supplying the extract to an aldol purification column and distilling the extract to obtain a lower discharge stream including hydroxypivaldehyde and an upper discharge stream including unreacted isobutyl aldehyde; supplying the upper discharge stream from the aldol purification column to the integrated recovery column and supplying the lower discharge stream from the aldol purification column to a hydrogenation reactor to perform a hydrogenation reaction to obtain a second reaction product including neopentyl glycol; recovering unreacted isobutyl aldehyde and the catalyst from an upper portion of the integrated recovery column; and obtaining neopentyl glycol from the second reaction product.

### [Advantageous Effects]

According to the method for preparing neopentyl glycol of the present invention, a catalyst salt produced after an aldol condensation reaction is reduced to a catalyst by a saponification reaction and the reduced catalyst is recovered, thereby efficiently reusing the catalyst of the aldol condensation reaction. Thus, neopentyl glycol may be economically prepared and environmental pollution may be reduced.

Furthermore, the present invention recovers the reduced catalyst together in an integrated recovery column for reusing unreacted isobutyl aldehyde during the aldol condensation reaction, thereby simplifying the process and managing the process highly efficiently, as compared with a case of providing a separate recovery means for recovering the reduced catalyst.

In addition, upper waste heat of the integrated recovery column is used in, for example, steam production, thereby using energy more efficiently.

### [Description of Drawings]

FIG. 1 is a process flow diagram showing a method for preparing neopentyl glycol according to an exemplary embodiment of the present invention.
FIG. 2 is a process flow diagram showing a method for preparing neopentyl glycol according to Comparative Example 1.
FIG. 3 is a process flow diagram showing a method for preparing neopentyl glycol according to Comparative Example 2.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

In the present invention, the term "stream" may refer to a fluid flow in a process, or may refer to a fluid itself flowing in a pipe. Specifically, the "stream" may refer to both a fluid itself flowing in a pipe connecting each device and a fluid flow. In addition, the fluid may refer to a gas or liquid, and a case in which a solid substance is included in the fluid is not excluded.

Meanwhile, in the devices such as an extraction column, a purification column, a distillation column, and a recovery column, unless otherwise particularly stated, a "lower portion" of the device refers to a point at a height of 95% to 100% from top to bottom of the device, specifically a lowest part (bottom). Likewise, unless otherwise particularly stated, an "upper portion" of the device refers to, a point at a height of 0% to 5% from the top to the bottom of the device, specifically the highest part (top).

Meanwhile, in the devices such as an extraction column, a purification column, a distillation column, and a recovery column in the present invention, unless otherwise particularly stated, an operating temperature of the device may refer to a lower temperature of the device. Likewise, unless otherwise particularly stated, an operating pressure of the device may refer to upper pressure of the device.

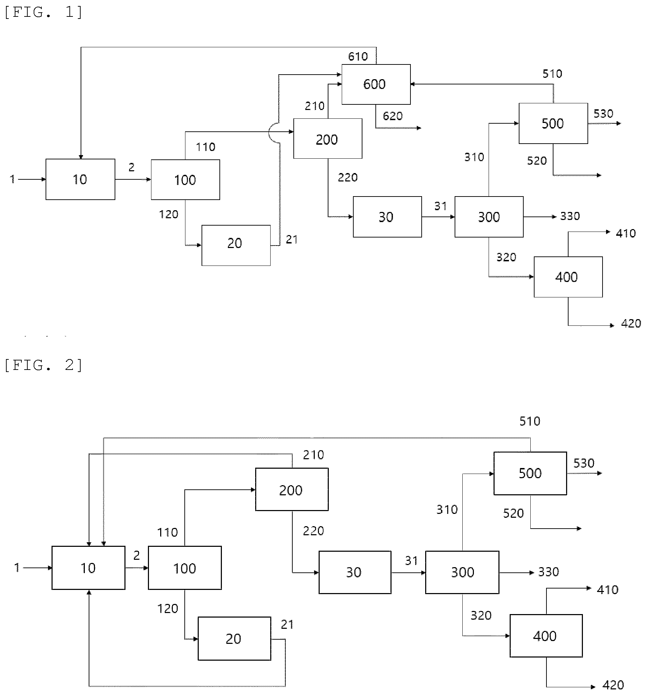

Hereinafter, the present invention will be described in more detail with reference to the FIG. 1 for better understanding of the present invention.

According to an exemplary embodiment of the present invention, provided is a method for preparing neopentyl glycol, the method including: performing an aldol condensation reaction of a formaldehyde aqueous solution and isobutyl aldehyde in the presence of a catalyst in an aldol reactor to obtain a first reaction product including hydroxypivaldehyde; supplying the first reaction product to an aldol extraction column and bringing the first reaction product into contact with an extractant to obtain an extract including hydroxypivaldehyde and a raffinate including a catalyst salt; supplying the raffinate to a saponification reactor to reduce the catalyst salt to the catalyst and supplying a discharge stream from the saponification reactor including the catalyst to an integrated recovery column; supplying the extract to an aldol purification column and distilling the extract to obtain a lower discharge stream including hydroxypivaldehyde and an upper discharge stream including unreacted isobutyl aldehyde; supplying the upper discharge stream from the aldol purification column to the integrated recovery column and supplying the lower discharge stream from the aldol purification column to a hydrogenation reactor to perform a hydrogenation reaction to obtain a second reaction product including neopentyl glycol (NPG); recovering unreacted isobutyl aldehyde and the catalyst from an upper portion of the integrated recovery column; and obtaining neopentyl glycol from the second reaction product.

First, the method for preparing neopentyl glycol according to an exemplary embodiment of the present invention may include performing an aldol condensation reaction of a formaldehyde aqueous solution and isobutyl aldehyde in the presence of a catalyst in an aldol reactor 10 to obtain a first reaction product including hydroxypivaldehyde.

The aldol condensation reaction may be performed by reacting a formaldehyde (FA) aqueous solution and isobutyl aldehyde (IBAL) in the presence of the catalyst in the aldol reactor 10. Specifically, a mixed solution including a formaldehyde aqueous solution and IBAL is supplied to an aldol reactor 10 as a feed stream 1, and the aldol condensation reaction is performed in the presence of the catalyst in the aldol reactor 10 to obtain a first reaction product including hydroxypivaldehyde (HPA).

Herein, formalin may be used as the formaldehyde aqueous solution, in which a concentration of the formalin of 35 to 45 wt% may be effective in terms of waste water reduction. The formaldehyde aqueous solution may include water in an amount of 40 to 64 wt%, more specifically 45 to 55 wt% with respect to the total weight of the formaldehyde aqueous solution, and may include methanol for preventing polymerization of formaldehyde. In this case, the amount of methanol may be 0.1 to 15 wt%, more specifically 0.1 to 5 wt% with respect to the total weight of formaldehyde aqueous solution. When the amount of methanol is less than 0.1 wt%, the amount of methanol in the formaldehyde aqueous solution is insufficient, so that a polycondensation reaction in which the aldol condensation reaction is repeated may be caused. When the amount of methanol is more than 15 wt%, the amount of methanol in the formaldehyde aqueous solution is raised and the concentration of formaldehyde may be excessively lowered.

Herein, the catalyst may be an amine-based compound. Specifically, a tertiary amine compound such as trialkylamine, trimethylamine, triethylamine, tripropylamine, triisopropylamine, and tributylamine may be appropriate. More specifically, the catalyst may include triethyl amine (TEA). In the present invention, since TEA is most efficient in the aldol condensation reaction, it may be used as the catalyst.

In the aldol reactor 10, an aldol condensation reaction temperature may be 70°C to 100°C. When the aldol condensation reaction temperature is lower than 70°C, the aldol condensation reaction may not be performed well due to the low temperature, and thus, it may be difficult to obtain the first reaction product including HPA with a high conversion rate. When the aldol condensation reaction temperature is higher than 100°C, production of by-products during the aldol condensation reaction may be accelerated.

A residence time in the aldol reactor 10 may be 0.1 hours to 3 hours. When the residence time is less than 0.1 hours, a HPA yield may be decreased according to the progress of the aldol condensation reaction, and when the residence time is more than 3 hours, as the aldol condensation reaction proceeds for a long time, energy efficiency is decreased and by-products may be excessively produced.

The aldol condensation reaction proceeds under the conditions described above in the aldol reactor 10 of the present invention, thereby producing HPA. Herein, formic acid is produced by a Cannizzaro side reaction occurring in the aldol condensation reaction, and the formic acid is reacted with TEA as a catalyst to produce a TEA salt, that is, a catalyst salt.

In addition, hydroxypivalic acid-neopentyl glycol ester (HPNE) may be produced by a Tishchenko reaction which is another side reaction of the aldol condensation reaction. Previously, it was common to treat the HPNE as a by-product and discard it, but according to an exemplary embodiment of the present invention, HPNE may be separated and used as a valuable raw material.

As a result, a stream 2 discharged from the aldol reactor may include HPA, HPNE, and a catalyst salt as the first reaction product.

Subsequently, the method for preparing neopentyl glycol according to an exemplary embodiment of the present invention may include supplying the first reaction product to an aldol extraction column 100 and bringing the first reaction product into contact with an extractant to obtain an extract including hydroxypivaldehyde and a raffinate including a catalyst salt.

More specifically, the first reaction product including HPA produced in the aldol reactor 10 may be supplied to the aldol extraction column 100 through an aldol reactor discharge stream 2. In the aldol extraction column 100, the first reaction product supplied through the aldol reactor discharge stream 2 may be brought into contact with an extractant to obtain an organic phase extract including HPNE, HPA, and an extractant, and a liquid raffinate including a catalyst salt. Herein, the catalyst salt may be present in a state of being dissociated in water, and the water may be derived from a formic acid aqueous solution.

Herein, the extractant may be aliphatic alcohols, and preferably, may include 2-ethylhexanol (2-EH). Since HPA included in the first reaction product is soluble in the 2-EH, it may be preferably used in the aldol extraction column 100 of the present invention which is an extraction device according to a liquid-liquid contact method described later.

An extraction device according to a liquid-liquid contact method may be used as the aldol extraction column 100. For example, the extraction device may be an extraction device such as a Karr type reciprocating plate column, a rotary-disk contactor, a Scheibel column, a spray extraction column, a packed extraction column, and a pulsed packed column.

In addition, the aldol extraction column 100 may reduce energy used in distillation in an aldol purification column 200 described later, by separating a large amount of water included in the aldol reactor discharge stream 2 as a raffinate. Herein, the water may be water included in the formaldehyde aqueous solution.

An operating temperature of the aldol extraction column 100 may be 40°C to 90°C. When the operating temperature is lower than 40°C, the first reaction product is not distilled and HPA included in the first reaction product may be cured. When the operating temperature is higher than 90°C, it may be difficult to perform phase separation of the first reaction product introduced to the aldol extraction column 100 into an organic phase and a liquid phase.

Meanwhile, according to an exemplary embodiment of the present invention, the extract may be supplied to the aldol purification column 200 as an extract stream 110. The extract may further include unreacted IBAL and a catalyst in addition to HPA and an extractant. Herein, the unreacted IBAL may be unreacted IBAL in the aldol condensation reaction performed in the aldol reactor 10. Meanwhile, the raffinate may be supplied to a saponification reactor 20 as a raffinate stream 120.

The method for preparing neopentyl glycol according to an exemplary embodiment of the present invention may include supplying the raffinate to the saponification reactor 20 to reduce the catalyst salt to the catalyst and supplying a discharge stream from the saponification reactor including the catalyst to an integrated recovery column 600.

Specifically, the catalyst salt included in the raffinate may be reduced to a catalyst by a saponification reaction in the saponification reactor 20. The catalyst salt may be formed by a side reaction by the aldol condensation reaction as described above.

The saponification reaction may be performed by a reaction of an inorganic strong base such as sodium hydroxide (NaOH) separately added and a catalyst salt, thereby efficiently reusing a reduced catalyst. For example, when TEA is used as a catalyst of the aldol condensation reaction, a salt of TEA may be reduced to TEA by the following Reaction Formula 1:

[Reaction Formula 1] TEA-Salt + NaOH → TEA + Na-Salt

Meanwhile, in the saponification reactor 20, a temperature of the saponification reaction may be 50°C or higher, 55°C or higher, or 60°C or higher and 90°C or lower, 95°C or lower, or 100°C or lower. When the saponification reaction temperature is lower than 50°C, the saponification reaction may not be performed well due to the low temperature, and a conversion rate to the catalyst may be lowered. When the saponification reaction temperature is higher than 100°C, by-products may be excessively produced during the saponification reaction.

When the catalyst salt is not reduced to the catalyst through the saponification reaction and supplied in a state of the catalyst salt to a hydrogenation reactor 30 in a latter stage described later, a hydrogenation reaction performed in the hydrogenation reactor may be adversely affected. Therefore, it is necessary to remove the catalyst salt in advance and recover the reduced catalyst. Specifically, under the conditions for the hydrogenation reaction in the hydrogenation reactor 30, the catalyst salt prevents the role of a hydrogenation reaction catalyst required for a hydrogenation reaction and causes various side reactions, and thus, the conversion rate of the hydrogenation reaction may be lowered.

Therefore, the catalyst salt is reduced to the catalyst in the saponification reactor 20 and the reduced catalyst is recovered in an integrated recovery column 600 described later, thereby preventing introduction of the catalyst salt to the hydrogenation reactor 30 to minimize various side effects described above.

In addition, the catalyst salt is reduced to the catalyst in the saponification reactor 20, thereby facilitating recovery of the catalyst in the integrated recovery column 600 described later, and thus, efficiently reusing the catalyst. Furthermore, an environmental pollution problem which may occur when the catalyst salt is discarded without reuse may be solved.

The catalyst reduced as such, for example, a saponification reactor discharge stream 21 including TEA may be then supplied to the integrated recovery column 600.

Meanwhile, the method for preparing neopentyl glycol according to an exemplary embodiment of the present invention may include supplying the extract to an aldol purification column and distilling the extract to obtain a lower discharge stream from the aldol purification column including hydroxypivaldehyde and an upper discharge stream from the aldol purification column including unreacted isobutyl aldehyde.

The extract described above may include the catalyst, unreacted isobutyl aldehyde (IBAL), HPNE, the extractant, and hydroxypivaldehyde (HPA). In addition, the extract is supplied to the aldol purification column 200 as an extract stream 110 and then distilled, and then may be separated into a lower fraction including HPA and an upper fraction including the catalyst and unreacted IBAL. Herein, the lower fraction may further include the extractant and HPNE.

Meanwhile, an operating temperature of the aldol purification column 200 may be 40°C or higher, 45°C or higher, 50°C or higher, or 55°C or higher and 85°C or lower, 90°C or lower, 95°C or lower, or 100°C or lower.

In addition, an operating pressure of the aldol purification column 200 may be 40.00 kPa(300 torr) or more, 46.66 kPa(350 torr) or more, 53.33 kPa(400 torr) or more, or 60.00 kPa(450 torr) or more and 80.00 kPa(600 torr) or less, 86.66 kPa(650 torr) or less, 93.33 kPa(700 torr) or less, or 101.32 kPa(760 torr) or less. By operating the aldol purification column 200 within the ranges of temperature and pressure, distillation is performed well, so that separation of TEA which is a relatively low-boiling point material and HPA which is a relatively high-boiling point material may be easily performed.

Therefore, the upper fraction of the aldol purification column 200 including unreacted isobutyl aldehyde may be supplied to the integrated recovery column 600 through an upper discharge stream 210 from the aldol purification column. Meanwhile, the lower fraction of the aldol purification column 200 including hydroxypivaldehyde may be supplied to a hydrogenation reactor 30 through a lower discharge stream 220 from the aldol purification column.

The method for preparing neopentyl glycol according to an exemplary embodiment of the present invention may include recovering unreacted isobutyl aldehyde and the catalyst from an upper portion of the integrated recovery column.

Specifically, in the integrated recovery column 600, the upper discharge stream 210 from the aldol purification column and the discharge stream 21 from the saponification reactor described above may be distilled. Specifically, an upper fraction including unreacted IBAL and the catalyst and a lower fraction including waste water, for example, water and other impurities (2-EH, unidentified by-products, MEOH, etc.) may be separated by the distillation. Herein, the upper fraction of the integrated recovery column may be circulated to the aldol reactor 10 as an upper discharge stream 610 from the integrated recovery column, and the lower fraction may be supplied to a separate waste water treatment process as a lower discharge stream 620 from the integrated recovery column.

Meanwhile, according to the present invention, in addition to the upper discharge stream 210 from the aldol purification column and the discharge stream 21 from the saponification reactor described above, an upper discharge stream 510 from an extractant purification column described layer may be further supplied to the integrated recovery column 600.

Conventionally, the upper discharge stream 210 from the aldol purification column and the discharge stream 21 from the saponification reactor are supplied to separate recovery columns, respectively, and unreacted IBAL and the catalyst are separated in the upper portion and water and by-products are separated in the lower portion.

However, according to the present invention, a recovery process of supplying the upper discharge stream 210 from the aldol purification column and the discharge stream 21 from the saponification reactor to one integrated recovery column to separate and recover unreacted IBAL and the catalyst (e.g., TEA) in the upper portion may be performed. That is, unreacted IBAL and TEA may be distilled by one integrated recovery column, not by applying separate recovery columns to each stream, since amounts of TEA included in the upper discharge stream 210 from the aldol purification column and the discharge stream 21 from the saponification reactor are similar and TEA and IBAL having a lower boiling point than TEA may be distilled simultaneously by one distillation column.

According to an exemplary embodiment of the present invention, a ratio of a catalyst amount in the discharge stream 21 from the saponification reactor to a catalyst amount in the upper discharge stream 210 from the aldol purification column including the upper fraction of the aldol purification column 200 may be 0.60 or more, 0.65 or more, or 0.70 or more and 1.10 or less, 1.15 or less, or 1.20 or less. By supplying a stream having a similar composition of the catalyst in the stream to the integrated recovery column 600, distillation and separation which are conventionally performed in separate recovery columns, respectively, may be performed in one recovery column.

Specifically, when a ratio of the catalyst content in the discharge stream 21 from the saponification reactor to the catalyst amount in the upper discharge stream 210 from the aldol purification column is less than 0.60, a flow rate of the lower discharge stream 120 from the aldol extraction column supplied to the saponification reactor 20 should be decreased, and thus, a flow rate of the upper discharge stream 110 from the aldol extraction column supplied to the aldol purification column 200 may be increased. Thus, energy usage in the aldol purification column 200 may be increased as the flow rate of the upper discharge stream 110 from the aldol extraction column is increased.

Meanwhile, when a ratio of the catalyst content in the discharge stream 21 from the saponification reactor to the catalyst amount in the upper discharge stream 210 from the aldol purification column is more than 1.20, a flow rate of the lower discharge stream 120 from the aldol extraction column supplied to the saponification reactor 20 should be increased, and thus, a flow rate of the upper discharge stream 110 from the aldol extraction column supplied to the aldol purification column 200 may be decreased. Therefore, energy usage in the aldol purification column 200 may be decreased as the flow rate of the upper discharge stream 110 from the aldol extraction column is decreased, but a large amount of HPA is introduced to the saponification reactor 20 as the flow rate of the lower discharge stream 120 from the aldol extraction column is increased, and thus, an yield of NPG by the hydrogenation reaction of HPA in the hydrogenation reactor 30 described later may be decreased. In addition, NPG may be introduced to the saponification reactor 20 in addition to the HPA to cause loss of NPG. Herein, the NPG is NPG which is not obtained from an NPG purification column 300 described later, but refluxed to the aldol reactor 10 through an extractant purification column 500 and an integrated recovery column 600.

More specifically, the catalyst is recovered together in the integrated recovery column 600 for reusing unreacted IBAL during the aldol condensation reaction, thereby simplifying the process as compared with the case of providing a separate recovery means for recovering the catalyst and reducing energy usage to manage the process highly efficiently.

Furthermore, upper waste heat of the integrated recovery column 600 is recovered by high pressure and high temperature conditions in the upper portion of the integrated recovery column to obtain a reuse effect also. The upper waste heat may be used by producing steam after heating cooled steam through a separate heat exchanger or supplied to a reboiler in the lower portion of the aldol purification column to be used in heating of the aldol purification column.

In order to obtain the effect, the upper portion of the integrated recovery column 600 may be at a height of 70% to 100%, specifically 75% to 100%, or 85% to 100% from the top to the bottom of the integrated recovery column, when the highest part (top) is 100%. By setting the upper portion of the integrated recovery column 600 to the height within the range, upper waste heat of the integrated recovery column 600 may be easily recovered under operating pressure and temperature conditions in the upper portion of the integrated recovery column 600 described later.

Herein, an operating pressure in the upper portion of the integrated recovery column may be 58.66 kPa(440 torr) or more, 98.00 kPa(735 torr) or more, 146.65 kPa(1100 torr) or more, or 193.32 kPa(1450 torr) or more and 293.31 kPa(2200 torr) or less, 333.31 kPa(2500 torr) or less, 393.30 kPa(2950 torr) or less, or 479.96 kPa(3600 torr) or less. By operating the upper portion of the integrated recovery column 600 at an operating pressure within the range, unreacted IBAL and the catalyst may be recovered separately from impurities, as described above, and also, a heat source removed in the upper portion of the integrated recovery column 600 may be reused to improve energy efficiency.

Specifically, when the operating pressure in the upper portion of the integrated recovery column 600 is less than 58.66 kPa(440 torr), it is difficult to recover both unreacted IBAL and the catalyst and it may be difficult to perform a function of the integrated recovery column 600 to be desired. When the operating pressure in the upper portion of the integrated recovery column is more than 479.96 kPa(3600 torr), operating energy therefor may be excessively consumed.

A temperature in the upper portion of the integrated recovery column may be 50°C or higher, 55°C or higher, 60°C or higher, or 65°C or higher and 135°C or lower, 140°C or lower, 145°C or lower, or 150°C or lower. When the operating temperature in the upper portion of the integrated recovery column 600 is lower than 50°C, it may be difficult to recover unreacted IBAL and the catalyst, and thus, the amounts of unreacted IBAL and the catalyst lost as waste water are increased, which may be disadvantageous in terms of process costs. In addition, when the operating temperature in the upper portion of the integrated recovery column is higher than 150°C, it may be difficult to recover energy from upper waste heat of the integrated recovery column 600.

Therefore, the upper discharge stream 610 from the integrated recovery column including unreacted IBAL and the catalyst which are simultaneously recovered from the upper portion of the integrated recovery column 600 may be circulated to the aldol reactor 10. More specifically, a part of the upper discharge stream 610 from the integrated recovery column may be circulated to the aldol reactor 10 through a condenser and the rest may be circulated to the integrated recovery column 600. By circulating the upper discharge stream 610 from the integrated recovery column including the catalyst and unreacted IBAL to the aldol reactor 10, the catalyst and IBAL used in the aldol reactor 10 are reused, and thus, a newly added amount may be reduced and production costs used during the process may be reduced.

The method for preparing neopentyl glycol according to an exemplary embodiment of the present invention may include supplying the lower discharge stream from the aldol purification column to a hydrogenation reactor 30 and performing a hydrogenation reaction to obtain a second reaction product including neopentyl glycol.

The lower discharge stream from the aldol purification column may include HPA and an extractant, and further include HPNE. A hydrogenation reaction may proceed in the hydrogenation reactor 30, and the hydrogenation reaction may be performed by a reaction of hydrogen which is further added to the hydrogenation reactor 30 and HPA in the presence of a hydrogenation reaction catalyst.

The hydrogenation reaction may proceed at a hydrogen pressure of 689.48 kPa to 10342.14 kPa (100 to 1500 psig, pounds per square inch gauge pressure) and a reaction temperature of 100°C to 200°C. In addition, a nickel catalyst or a copper-based catalyst may be used as the hydrogenation reaction catalyst. The copper-based catalyst may be, for example, a CuO/BaO/SiO catalyst, and the CuO/BaO/SiO catalyst may be a catalyst of (CuO)ₓ(BaO)_{y}(SiO)_{z} (x, y, and z are wt%, and x:y:z = x:y:z=10-50:0-10:40-90, 10-50:1-10:40-89, or 29-50:1-10:40-70). The sum of x and y is preferably 20 to 50 (wt%) or 30 to 50 (wt%), based on the sum of x, y, and z (100 wt%), and in this range, performance of the hydrogenation reaction catalyst is excellent and an effect of long life is shown. Meanwhile, the nickel catalyst may be 2 to 10 wt% with respect to the weight of HPA.

As described above, when a catalyst salt formed by the aldol condensation reaction, for example, a TEA salt, is introduced to the hydrogenation reaction, various side reactions may be caused to decrease a conversion rate of the hydrogenation reaction. Therefore, TEA converted by the saponification reactor 20 of the present invention is recovered in the integrated recovery column 600, thereby improving the conversion rate of the hydrogenation reaction in the hydrogenation reactor 30.

As such, the hydrogenation reaction proceeds in the hydrogenation reactor 30, and when HPA reacts with hydrogen, NPG may be produced. Therefore, after the hydrogenation reaction, a second reaction product including the catalyst, the extractant, HPNE, and the NPG may be obtained. Subsequently, a desired product, neopentyl glycol, may be obtained by the purification process of the second reaction product.

Specifically, the purification process of the second reaction product may be performed by including: supplying the second reaction product to a neopentyl glycol purification column 300 to supply a stream including the catalyst and the extractant to an extractant purification column 500, supplying a stream including hydroxypivalic acid-neopentyl glycol ester to a hydroxypivalic acid-neopentyl glycol ester purification column 400, and obtaining neopentyl glycol from a stream including neopentyl glycol; and obtaining hydroxypivalic acid-neopentyl glycol ester from the lower portion of the hydroxypivalic acid-neopentyl glycol ester purification column.

Specifically, the second reaction product may be supplied to a neopentyl glycol (NPG) purification column 300 as a discharge stream 31 from the hydrogenation reactor. Herein, the NPG purification column 300 may be one or two or more purification columns.

First, when the NPG purification column 300 is one purification column, the extractant and the catalyst may be separated from an upper portion, HPNE may be separated from a lower portion, and NPG may be separated from a side portion of the one purification column, respectively. For example, the NPG purification column 300 may be one separation wall-type distillation column. Herein, the NPG may be obtained from the side portion at a height of 40% to 80% from the top to the bottom of the NPG purification column 300. Meanwhile, when the NPG purification column 300 is two or more purification columns, it may be performed through one or more NPG purification columns which separate the catalyst into the upper portion and one or more NPG purification columns which separate the extractant into the upper portion. A stream including HPNE or NPG may be separated and discharged into the lower portion of two or more NPG purification columns.

That is, the second reaction product may be separated into the catalyst and the extractant, HPNE, and NPG by the one or two or more NPG purification columns 300. Thus, high-purity NPG may be obtained. A stream 310 including the catalyst and the extractant may be supplied to the extractant purification column 500, and a stream 320 including HPNE may be supplied to the HPNE purification column 400. To this end, an operating temperature of the NPG purification column 300 may be 80°C or higher, 100°C or higher, 120°C or higher, or 140°C or higher and 185°C or lower, 190°C or lower, 195°C or lower, or 200°C or lower. In addition, an operating pressure of the NPG purification column 300 may be 5.33 kPa(40 torr) or more, 11.00 kPa(90 torr) or more, 15.00 kPa(120 torr) or more, or 18.67 kPa(140 torr) or more and 40.00 kPa(300 torr) or less, 53.33 kPa(400 torr) or less, 66.66 kPa(500 torr) or less, or 80.00 kPa(600 torr) or less. By operating the NPG purification column 300 within the temperature and pressure ranges, it may be easy to separate the extractant and the catalyst, NPG, and HPNE, as described above. Therefore, since the amount of by-products present in a side discharge stream 330 from the NPG purification column including NPG to be obtained in the present invention may be decreased, high-purity NPG may be obtained.

Meanwhile, as a part of the HPNE produced by the side reaction of the aldol condensation reaction is reduced to NPG in the hydrogenation reactor 30 and the rest remains as HPNE, the HPNE is introduced to the NPG purification column 300 to decrease an NPG yield. Therefore, the HPNE is supplied to a HPNE purification column 400 described later, thereby further obtaining NPG which is not recovered in the NPG purification column 300, and also recovering the HPNE which is a high value-added product itself and using it. That is, it may be distinguished from other heavy by-products, for example, trimethylpentanediol (2,2,4-trimethyl-1,3-pentanediol; TMPD) which is a by-product of the hydrogenation reaction and be commercialized.

Meanwhile, obtaining of HPNE from the lower portion of the HPNE purification column described above may be performed specifically by distilling the stream 320 including HPNE in the HPNE purification column 400 to separate a lower fraction including HPNE and an upper fraction including NPG.

That is, since NPG which is not recovered in the NPG purification column 300 and discharged may be recovered from the upper portion (410) of the HPNE purification column 400, an NPG recovery rate may be further increased as compared with a conventional method for preparing NPG which is not provided with the HPNE purification column 400. In addition, since HPNE may be recovered from the lower fraction (420) of the HPNE purification column 400 and HPNE may be used as a raw material of other processes, for example, a main raw material of synthesis and coating of polyester, as described above, economic feasibility may be improved in terms of raw material utilization by the HPNE purification column 400 according to the present invention. Meanwhile, according to an exemplary embodiment of the present invention, the method for preparing neopentyl glycol may include: recovering a catalyst from the upper portion of the extractant purification column and supplying the catalyst to the integrated recovery column; and recovering the extractant from the lower portion of the extractant purification column.

Specifically, the stream 310 including the catalyst and the extractant may be distilled in the extractant purification column 500 and separated into an upper fraction including the catalyst, a lower fraction including the extractant, and a side fraction including waste oil, respectively. The waste oil included in the side fraction of the extractant purification column 500 may be discharged out of the system through a waste oil discharge stream 530.

Herein, the catalyst included in the upper fraction of the extractant purification column 500 may be some small amount of the catalyst which is not separated in the aldol extraction column 100 and the aldol purification column 200 described above. In the extractant purification column 500, the catalyst is separated and supplied to the integrated recovery column 600 through the upper discharge stream 510 from the extractant purification column, thereby recovering the catalyst in the system, for example, TEA.

Meanwhile, the lower fraction of the extractant purification column 500 including the extractant is discharged as a lower discharge stream 520 from the extractant purification column, and the extractant, for example, 2-EH may be separated and recovered therefrom and reused in the process in the former stage.

Hereinafter, the present invention will be described in more detail by the examples. However, the following examples are provided for illustrating the present invention.

### Examples

### Example 1

According to the process flow illustrated in FIG. 1, a preparation process of neopentyl glycol (NPG) was simulated, using an Aspen Plus simulator available from Aspen.

Specifically, an aldol condensation reaction of a formaldehyde aqueous solution and isobutyl aldehyde was performed in the presence of a catalyst (triethylamine, TEA) in an aldol reactor 10 to obtain a first reaction product 2 including hydroxypivaldehyde (HPA). At this time, the aldol condensation reaction was performed at a temperature of 85°C.

The first reaction product was supplied to the aldol extraction column 100 and brought into contact with an extractant (2-ethylhexanol; 2-EH) to obtain an extract including HPA and a raffinate including a catalyst salt.

A raffinate stream 120 including the raffinate was supplied to a saponification reactor 20, the catalyst salt was reacted with sodium hydroxide (NaOH) to be reduced to the catalyst, and a discharge stream 21 from the saponification reactor including the catalyst was supplied to an integrated recovery column 600. At this time, a saponification reaction performed in the saponification reactor 20 was performed at a temperature of 83°C.

Meanwhile, an extract stream 110 including the extract was supplied to the aldol purification column 200 and distilled to obtain a lower discharge stream 220 from the aldol purification column including HPA and an upper discharge stream 210 from the aldol purification column including unreacted isobutyl aldehyde (IBAL) and the catalyst. The operating pressure of the aldol purification column 200 was 27.60 kPa(207 torr) and the operating temperature thereof was 87°C. At this time, a ratio of the catalyst content in the discharge stream from the saponification reactor to the catalyst amount in the upper discharge stream from the aldol purification column was 0.9.

Subsequently, the upper discharge stream 210 from the aldol purification column was supplied to the integrated recovery column 600. In the integrated recovery column 600, streams 21, 210, and 510 supplied to the integrated recovery column were distilled, and were separated into an upper fraction including unreacted IBAL and the catalyst and a lower fraction including waste water.

The upper fraction of the integrated recovery column 600 was circulated to the aldol reactor 10 as an upper discharge stream 610 from the integrated recovery column. The lower fraction of the integrated recovery column was discharged out of the system as a lower discharge stream 620 from the integrated recovery column.

Meanwhile, a lower discharge stream 220 from the aldol purification column was supplied to a hydrogenation reactor 30 and hydrogenated to obtain a second reaction product including NPG. At this time, the hydrogenation reaction was performed at a temperature of 160°C.

The second reaction product was supplied to an NPG purification column 300, an upper discharge stream 310 from the NPG purification column including the catalyst and the extractant was supplied to an extractant purification column 500, and a lower discharge stream 320 from the NPG purification column including HPNE was supplied to the HPNE purification column 400, thereby obtaining NPG from the side discharge stream 330 from the NPG purification column. At this time, the operating pressure of the NPG purification column 300 was 20.53 kPa(154 torr) and the operating temperature thereof was 168°C.

At this time, the upper discharge stream 310 from the NPG purification column was distilled in the extractant purification column 500, and an upper discharge stream 510 from the extractant purification column including the catalyst was supplied to the integrated recovery column 600 and the extractant was separately obtained from a lower discharge stream 520 from the extractant purification column including the extractant.

Meanwhile, the lower discharge stream 320 from the NPG purification column was distilled in the HPNE purification column 400, and NPG was recovered from an upper discharge stream 410 from the HPNE purification column including NPG and HPNE was recovered from a lower discharge stream 420 from the HPNE purification column including HPNE.

### Example 2

In Example 2, NPG was prepared by the same process flow as in Example 1, except that a ratio of the catalyst content in the discharge stream from the saponification reactor to the catalyst amount in the upper discharge stream from the aldol purification column was 0.5.

### Example 3

In Example 3, NPG was prepared by the same process flow as in Example 1, except that a ratio of the catalyst content in the discharge stream from the saponification reactor to the catalyst amount in the upper discharge stream from the aldol purification column was 1.3.

### Comparative Examples

### Comparative Example 1

According to the process flow illustrated in FIG. 2, a preparation process of neopentyl glycol (NPG) was simulated, using the Aspen Plus simulator available from Aspen.

In Comparative Example 1, NPG was prepared in the same process flow as in Example 1, except that the discharge stream 21 from the saponification reactor, the upper discharge stream 210 from the aldol purification column, and the upper discharge stream 510 from the extractant purification column were supplied directly to the aldol reactor 10, not to the integrated recovery column.

### Comparative Example 2

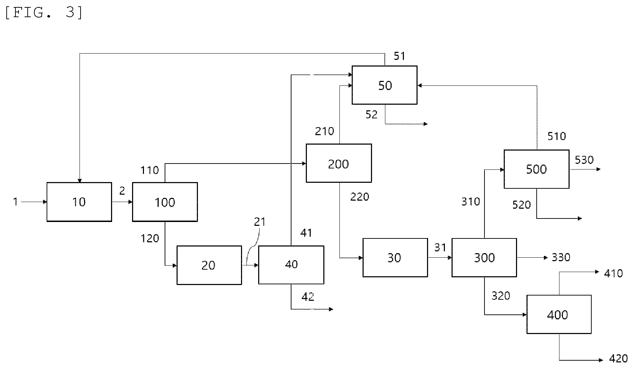

According to the process flow illustrated in FIG. 3, a preparation process of neopentyl glycol (NPG) was simulated, using the Aspen Plus simulator available from Aspen.

In Comparative Example 1, the discharge stream 21 from the saponification reactor was supplied to the catalyst recovery column 40 and distilled to be separated into an upper fraction including the catalyst and a lower fraction including water and by-products. The upper fraction including the catalyst was supplied to a raw material recovery column 50 through an upper discharge stream 41 from the catalyst recovery column. The lower fraction including water and by-products was discharged out of the system through the lower discharge stream 42 from the catalyst recovery column.

Meanwhile, the upper discharge stream 510 from the extractant purification column and the upper discharge stream 210 from the aldol purification column were supplied to the raw material recovery column 50. In the raw material recovery column 50, the streams 41, 210, and 510 supplied to the raw material recovery column were distilled to obtain an upper discharge stream 51 from the raw material recovery column including unreacted IBAL and the catalyst and a lower discharge stream 52 from the raw material recovery column including water and by-products.

NPG was prepared by the same process flow as in Example 1, except that the upper discharge stream 51 from the raw material recovery column was supplied to the aldol reactor 10 and the lower discharge stream 52 from the raw material recovery column was discharged out of the system.

In the following Table 1, the catalyst amount ratios, the number of recovery process columns, the number of recovery process heat exchangers, the recovery process energy use rates, and the NPG yields of the examples and the comparative examples are shown.

Specifically, the recovery process was performed by the integrated recovery column in the examples, and was performed by the catalyst recovery column and the raw material recovery column in Comparative Example 2. Accordingly, the number of columns of the recovery process and the number of heat exchangers are shown in the following Table 1.

In addition, the energy use rate of the recovery process is shown as the amount of energy used in the integrated recovery column of each example as compared with the total amount of energy used in the catalyst recovery column and the raw material recovery column of Comparative Example 2, as a percentage.

Meanwhile, the NPG yield is shown as the amount of NPG obtained in the NPG purification column of each of the examples and the comparative examples compared with the amount of NPG obtained in the NPG purification column of Example 1, as a percentage.

**[Table 1]**

| | Catalyst content ratio¹⁾ | Number of columns of recovery process | Number of heat exchangers of Recovery process | Energy use rate of recovery process (%) | MeOH removal | NPG yield (wt%) |
|---|---|---|---|---|---|---|
| Example 1 | 0.9 | 1 | 2 | 85.6 | ○ | 100 |
| Example 2 | 0.5 | 1 | 2 | 94.2 | ○ | 100 |
| Example 3 | 1.3 | 1 | 2 | 75 | ○ | 99 or less |
| Comparative Example 1 | 0.9 | - | - | - | × | 100 |
| Comparative Example 2 | 0.9 | 2 | 4 | 100 | ○ | 100 |
| ¹⁾ The catalyst content ratio shows a ratio of the catalyst content in the discharge stream from the saponification reactor to the catalyst content in the upper discharge stream from the aldol purification column. | | | | | | |

Referring to Table 1, each of the energy use rates of the recovery process of the examples is converted and shown, based on the total energy use amount of 100% in the recovery process (catalyst recovery column and raw material recovery column) of Comparative Example 2. It was confirmed that the examples had good recovery process energy use rates as compared with those of the comparative examples.

Meanwhile, it was confirmed that in Comparative Example 2, in which the catalyst recovery column and the raw material recovery column were provided, respectively, unlike the examples, the recovery process energy use rate was the highest.

## Claims

1. A method for preparing neopentyl glycol, the method comprising:
performing an aldol condensation reaction of a formaldehyde aqueous solution and isobutyl aldehyde in the presence of a catalyst in an aldol reactor to obtain a first reaction product including hydroxypivaldehyde;
supplying the first reaction product to an aldol extraction column and bringing the first reaction product into contact with an extractant to obtain an extract including hydroxypivaldehyde and a raffinate including a catalyst salt;
supplying the raffinate to a saponification reactor to reduce the catalyst salt to the catalyst and supplying a discharge stream from the saponification reactor including the catalyst to an integrated recovery column;
supplying the extract to an aldol purification column and distilling the extract to obtain a lower discharge stream from the aldol purification column including hydroxypivaldehyde and an upper discharge stream from the aldol purification column including unreacted isobutyl aldehyde;
supplying the upper discharge stream from the aldol purification column to the integrated recovery column and supplying the lower discharge stream from the aldol purification column to a hydrogenation reactor to perform a hydrogenation reaction to obtain a second reaction product including neopentyl glycol;
recovering unreacted isobutyl aldehyde and the catalyst from an upper portion of the integrated recovery column; and
obtaining neopentyl glycol from the second reaction product.

2. The method for preparing neopentyl glycol of claim 1, further comprising: circulating the unreacted isobutyl aldehyde and the catalyst recovered from the upper portion of the integrated recovery column to the aldol reactor.

3. The method for preparing neopentyl glycol of claim 1, wherein a ratio of a catalyst content in the discharge stream from the saponification reactor to a catalyst content in the upper discharge stream from the aldol purification column is 0.60 to 1.20.

4. The method for preparing neopentyl glycol of claim 1, wherein an operating pressure of the upper portion of the integrated recovery column is 58.66 kPa(440 torr) to 479.96 kPa (3600 torr).

5. The method for preparing neopentyl glycol of claim 1, wherein a temperature of the upper portion of the integrated recovery column is 50°C to 150°C.

6. The method for preparing neopentyl glycol of claim 1,
wherein the second reaction product includes the catalyst, neopentyl glycol, the extractant, and hydroxypivalic acid-neopentyl glycol ester (HPNE), and
the obtaining of neopentyl glycol from the second reaction product further includes:
supplying the second reaction product to a neopentyl glycol purification column, supplying a stream including the catalyst and the extractant to an extractant purification column, a stream including hydroxypivalic acid-neopentyl glycol ester to a hydroxypivalic acid-neopentyl glycol ester purification column, and obtaining neopentyl glycol from a stream including neopentyl glycol; and
obtaining hydroxypivalic acid-neopentyl glycol ester from a lower portion of the hydroxypivalic acid-neopentyl glycol ester purification column.

7. The method for preparing neopentyl glycol of claim 6, wherein the neopentyl glycol is obtained from a side portion at a height of 40% to 80% from the top to the bottom of the neopentyl glycol purification column.

8. The method for preparing neopentyl glycol of claim 6, which includes recovering the catalyst from an upper portion of the extractant purification column and supplying the catalyst to the integrated recovery column, and
recovering the extractant from a lower portion of the extractant purification column.

9. The method for preparing neopentyl glycol of claim 1, wherein the catalyst includes triethyl amine (TEA).

10. The method for preparing neopentyl glycol of claim 1, wherein the extractant includes 2-ethyl hexanol (2-EH).

11. The method for preparing neopentyl glycol of claim 4, wherein the upper portion of the integrated recovery column is at 70% to 100% from the top to the bottom of the integrated recovery column.

## Patentansprüche

1. Verfahren zur Herstellung von Neopentylglykol, wobei das Verfahren umfasst:
Durchführen einer Aldolkondensationsreaktion einer wässrigen Formaldehydlösung und von Isobutylaldehyd in Gegenwart eines Katalysators in einem Aldolreaktor, um ein erstes Reaktionsprodukt zu erhalten, das Hydroxypivaldehyd enthält;
Zuführen des ersten Reaktionsprodukts zu einer Aldolextraktionssäule und Inkontaktbringen des ersten Reaktionsprodukts mit einem Extraktionsmittel, um einen Extrakt, der Hydroxypivaldehyd enthält, und ein Raffinat, das ein Katalysatorsalz enthält, zu erhalten;
Zuführen des Raffinats zu einem Verseifungsreaktor, um das Katalysatorsalz zu dem Katalysator zu reduzieren, und Zuführen eines Austragsstroms aus dem Verseifungsreaktor, der den Katalysator enthält, zu einer integrierten Rückgewinnungssäule;
Zuführen des Extrakts zu einer Aldolreinigungssäule und Destillieren des Extrakts, um einen unteren Austragsstrom aus der Aldolreinigungssäule, der Hydroxypivaldehyd enthält, und einen oberen Austragsstrom aus der Aldolreinigungssäule, der nicht umgesetzten Isobutylaldehyd enthält, zu erhalten;
Zuführen des oberen Austragsstroms aus der Aldolreinigungssäule zu der integrierten Rückgewinnungssäule und Zuführen des unteren Austragsstroms aus der Aldolreinigungssäule zu einem Hydrierungsreaktor, um eine Hydrierungsreaktion durchzuführen, um ein zweites Reaktionsprodukt zu erhalten, das Neopentylglykol enthält;
Rückgewinnen von nicht umgesetztem Isobutylaldehyd und des Katalysators aus einem oberen Abschnitt der integrierten Rückgewinnungssäule; und
Erhalten von Neopentylglykol aus dem zweiten Reaktionsprodukt.

2. Verfahren zur Herstellung von Neopentylglykol nach Anspruch 1, ferner umfassend: Zirkulieren des nicht umgesetzten Isobutylaldehyds und des Katalysators, die aus dem oberen Abschnitt der integrierten Rückgewinnungssäule zurückgewonnen wurden, zu dem Aldolreaktor.

3. Verfahren zur Herstellung von Neopentylglykol nach Anspruch 1, wobei ein Verhältnis eines Katalysatorgehalts in dem Austragsstrom aus dem Verseifungsreaktor zu einem Katalysatorgehalt in dem oberen Austragsstrom aus der Aldolreinigungssäule 0,60 bis 1,20 beträgt.

4. Verfahren zur Herstellung von Neopentylglykol nach Anspruch 1, wobei ein Betriebsdruck des oberen Abschnitts der integrierten Rückgewinnungssäule 58,66 kPa (440 Torr) bis 479,96 kPa (3600 Torr) beträgt.

5. Verfahren zur Herstellung von Neopentylglykol nach Anspruch 1, wobei eine Temperatur des oberen Abschnitts der integrierten Rückgewinnungssäule 50 °C bis 150 °C beträgt.

6. Verfahren zur Herstellung von Neopentylglykol nach Anspruch 1,
wobei das zweite Reaktionsprodukt den Katalysator, Neopentylglykol, das Extraktionsmittel und Hydroxypivalinsäure-Neopentylglykolester (HPNE) enthält, und
das Erhalten von Neopentylglykol aus dem zweiten Reaktionsprodukt ferner umfasst:
Zuführen des zweiten Reaktionsprodukts zu einer Neopentylglykolreinigungssäule, Zuführen eines Stroms, der den Katalysator und das Extraktionsmittel enthält, zu einer Extraktionsmittelreinigungssäule, eines Stroms, der Hydroxypivalinsäure-Neopentylglykolester enthält, zu einer Hydroxypivalinsäure-Neopentylglykolesterreinigungssäule und Erhalten von Neopentylglykol aus einem Strom, der Neopentylglykol enthält; und
Erhalten von Hydroxypivalinsäure-Neopentylglykolester aus einem unteren Abschnitt der Hydroxypivalinsäure-Neopentylglykolesterreinigungssäule.

7. Verfahren zur Herstellung von Neopentylglykol nach Anspruch 6, wobei das Neopentylglykol aus einem Seitenabschnitt in einer Höhe von 40 % bis 80 % von der Oberseite zur Unterseite der Neopentylglykolreinigungssäule erhalten wird.

8. Verfahren zur Herstellung von Neopentylglykol nach Anspruch 6, das das Rückgewinnen des Katalysators aus einem oberen Abschnitt der Extraktionsmittelreinigungssäule und das Zuführen des Katalysators zu der integrierten Rückgewinnungssäule und
das Rückgewinnen des Extraktionsmittels aus einem unteren Abschnitt der Extraktionsmittelreinigungssäule umfasst.

9. Verfahren zur Herstellung von Neopentylglykol nach Anspruch 1, wobei der Katalysator Triethylamin (TEA) enthält.

10. Verfahren zur Herstellung von Neopentylglykol nach Anspruch 1, wobei das Extraktionsmittel 2-Ethylhexanol (2-EH) enthält.

11. Verfahren zur Herstellung von Neopentylglykol nach Anspruch 4, wobei der obere Abschnitt der integrierten Rückgewinnungssäule 70 % bis 100 % von der Oberseite zu der Unterseite der integrierten Rückgewinnungssäule beträgt.

## Revendications

1. Procédé de préparation de néopentylglycol, le procédé comprenant:
la réalisation d'une réaction de condensation aldolique d'une solution aqueuse de formaldéhyde et d'isobutylaldéhyde en présence d'un catalyseur dans un réacteur aldolique afin d'obtenir un premier produit de réaction contenant de l'hydroxypivaldéhyde;
introduire le premier produit de réaction dans une colonne d'extraction aldolique et mettre le premier produit de réaction en contact avec un agent d'extraction pour obtenir un extrait comprenant de l'hydroxypivaldéhyde et un raffinat contenant un sel catalyseur;
fournir le raffinat à un réacteur de saponification pour réduire le sel catalytique en catalyseur et fournir un flux de décharge provenant du réacteur de saponification contenant le catalyseur à une colonne de récupération intégrée;
fournir l'extrait à une colonne de purification aldolique et distiller l'extrait pour obtenir un flux de décharge inférieur provenant de la colonne de purification aldolique contenant de l'hydroxypivaldéhyde et un flux de décharge supérieur provenant de la colonne de purification aldolique comprenant de l'isobutylaldéhyde n'ayant pas réagi;
fournir le flux de décharge supérieur provenant de la colonne de purification aldolique à la colonne de récupération intégrée et fournir le flux de décharge inférieur provenant de la colonne de purification aldolique à un réacteur d'hydrogénation afin d'effectuer une réaction d'hydrogénation pour obtenir un deuxième produit de réaction comprenant du néopentylglycol;
récupérer l'isobutylaldéhyde n'ayant pas réagi et le catalyseur à partir d'une partie supérieure de la colonne de récupération intégrée; et
obtenir du néopentylglycol à partir du deuxième produit de réaction.

2. Procédé de préparation de néopentylglycol selon la revendication 1, comprenant en outre: la circulation de l'isobutylaldéhyde n'ayant pas réagi et du catalyseur récupérés à partir de la partie supérieure de la colonne de récupération intégrée vers le réacteur aldolique.

3. Procédé de préparation de néopentylglycol selon la revendication 1, dans lequel le rapport entre la teneur en catalyseur dans le flux de décharge du réacteur de saponification et la teneur en catalyseur dans le flux de décharge supérieur de la colonne de purification aldolique est compris entre 0,60 et 1,20.

4. Procédé de préparation du néopentylglycol selon la revendication 1, dans lequel la pression de fonctionnement de la partie supérieure de la colonne de récupération intégrée est comprise entre 58,66 kPa (440 torr) et 479,96 kPa (3600 torr).

5. Procédé de préparation du néopentylglycol selon la revendication 1, dans lequel la température de la partie supérieure de la colonne de récupération intégrée est comprise entre 50 °C et 150 °C.

6. Procédé de préparation du néopentylglycol selon la revendication 1,
dans lequel le deuxième produit de réaction contient le catalyseur, le néopentylglycol, l'agent d'extraction et l'ester d'hydroxypivalique et de néopentylglycol (HPNE), et
l'obtention du néopentylglycol à partir du deuxième produit de réaction contient en outre:
l'alimentation d'une colonne de purification de néopentylglycol avec le deuxième produit de réaction, l'alimentation d'une colonne de purification d'extractant avec un flux contenant le catalyseur et l'extractant, l'alimentation d'une colonne de purification d'ester d'acide hydroxypivalique-néopentylglycol avec un flux contenant de l'ester d'acide hydroxypivalique-néopentylglycol, et l'obtention de néopentylglycol à partir d'un flux comprenant du néopentylglycol; et
l'obtention d'ester d'acide hydroxypivalique-néopentylglycol à partir d'une partie inférieure de la colonne de purification d'ester d'acide hydroxypivalique-néopentylglycol.

7. Procédé de préparation du néopentylglycol selon la revendication 6, dans lequel le néopentylglycol est obtenu à partir d'une partie latérale située à une hauteur comprise entre 40 % et 80 % entre le haut et le bas de la colonne de purification du néopentylglycol.

8. Procédé de préparation du néopentylglycol selon la revendication 6, qui comprend la récupération du catalyseur à partir d'une partie supérieure de la colonne de purification de l'agent d'extraction et l'alimentation du catalyseur vers la colonne de récupération intégrée, et
la récupération de l'agent d'extraction à partir d'une partie inférieure de la colonne de purification de l'agent d'extraction.

9. Procédé de préparation de néopentylglycol selon la revendication 1, dans lequel le catalyseur comprend de la triéthylamine (TEA).

10. Procédé de préparation du néopentylglycol selon la revendication 1, dans lequel l'agent d'extraction comprend du 2-éthylhexanol (2-EH).

11. Procédé de préparation du néopentylglycol selon la revendication 4, dans lequel la partie supérieure de la colonne de récupération intégrée se situe entre 70 % et 100 % de la hauteur totale de la colonne de récupération intégrée.
